# EUROPEAN PATENT SPECIFICATION

(11) **EP 0 548 087 B1**
(45) Date of publication and mention of the grant of the patent: **23.10.1996**
(21) Application number: 91912522.9
(22) Date of filing: 27.06.1991
(51) Int. Cl.: C08F 2/50, C08F 216/12, C09D 4/00, C08F 224/00

(54) **RADIATION CURABLE COATING COMPOSITIONS**
DURCH STRAHLUNG HÄRTBARE BESCHICHTUNGSZUSAMMENSETZUNGEN
COMPOSITIONS POUR REVETEMENT A POLYMERISATION INDUITE PAR RAYONNEMENT

(30) Priority: 10.09.1990 US 579512; 10.09.1990 US 582033
(43) Date of publication of application: 30.06.1993
(73) Proprietor: ISP INVESTMENTS INC., Wilmington,Delaware 19801 (US)
(72) Inventor: NARAYANAN, Kolazi, S., Palisades Park, NJ 07650 (US); PLOTKIN, Jeffrey, S., Monsey, NY 10952 (US); VARA, Fulvio, J., Chester, NJ 07930 (US); DOUGHERTY, James, A., Pequannock, NJ 07440 (US); MILLER, Mark, M., Ridgewood, NJ 07450 (US); TAYLOR, Paul, D., West Milford, NJ 07489 (US)
(74) Representative: Ford, Michael Frederick
(86) International application number: US9104619
(87) International publication number: WO9204383

(56) References cited:
- US-A- 2 522 680
- US-A- 3 249 631
- US-A- 3 422 118
- US-A- 3 532 715
- US-A- 4 108 747
- US-A- 4 634 722
- US-A- 4 885 319
- DERWENT ABSTRACT No. 76-31371X/17 05 May 1976.
- CHEMICAL ABSTRACTS, Vol. 82, No. 1 (1975) page 368, Abstract No. 4231s.

## Description

A cationically induced radiation polymerizable composition comprises a mixture of (a) between about 1 and about 75 wt. % of a reactive vinyl or alk-I-enyl ether cyclocarbonate having the formula 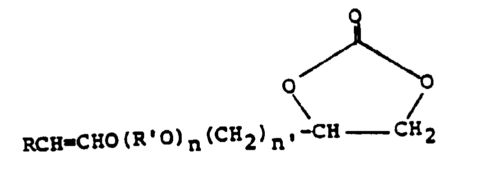 wherein R is hydrogen or lower alkyl; R' is C₂ to C₄ alkylene; n has a value of from 0 to 4 and n' has a value of from 1 to 4 and (b) between 99 and 25 wt. % of a coreactant monomer and/or oligomer which mixture additionally contains between 0.5 and 5% of a cationic onium salt initiator.

Component (b) of the composition can be a monomer or oligomer in which an onium salt is normally insoluble or sparingly soluble such as vinyl ether monomer or oligomer$)alkyl monovinyl ethers or alkyl monoalk-I-enyl ethers, the divinyl ether of cyclohexane dimethanol a vinyl ether capped urethane or an ester and the like.

Component (b) can also be a monomer or oligomer of an epoxide, e.g. bisphenol A diepoxide, a Novolac resin, and the like. Acrylates or methacrylates can also be employed as component (b) alone or in admixture with any of the aforementioned comonomers; however, these comonomers require a hybrid curing system of onium salt and free radical initiator.

The preferred compositions of the present invention are those wherein n of component (a) has a value of zero, n' has a value of one and R is hydrogen or methyl and component (b) is a divinyl ether terminated compound, most preferably, dimethyl cyclohexyl divinyl ether, mixed with a vinyl ether or epoxide capped oligomer The mole ratio of component (a) to component (b) in the mixture can vary within a wide range; however, an excess of component (b), e.g. a ratio of between about 1:20 and about 1:2 is recommended.

It has been found that the present compounds of component (a), having a vinyl or alk-1-enyl ether terminal group bonded directly or indirectly to the alkyl cyclocarbonate group, provide excellent solubilizing properties for the cationic initiators resulting in stable liquid initiator containing solutions. Also the vinyl or alk-l-enyl ether terminated cyclocarbonates are capable of interacting with polymerizable monomers and oligomers of component (b) to form non-yellowing, branched coatings of high resistance to abrasion and chemical attack. Unsubstituted cyclocarbonates and alkyl or polyether substituted cyclocarbonates do not share these properties since they fail to react with polymerizable monomers and oligomers. It has also been found that polyether compounds, which have been used to solubilize onium initiators, cause yellowing in the finished product and are therefore objectionable for many uses. The present vinyl or alk-I-enyl ether cyclocarbonates are superior solubilizers, do not cause yellowing and can be used in much smaller amounts to provide a stable liquid initiator solution. However, because the vinyl or alk-l-enyl ether cyclocarbonates of the present invention are multifunctional, i.e. excellent solvents for the difficultly solubilizable cationic initiators and reactive monomers with the monomers or oligomers of component (b) they can be used in substantially large amounts to alter properties in the cured product and to replace the conventional non-reactive initiator solvents, e.g. butyrolactone or propylene carbonate, and other solvents commonly employed in prior cationic systems. Prior initiator solvents were generally employed in low concentrations since their non-polymerizable nature causes tacky areas and discontinuous coating or blistering which can lead to rupture in the finally cured coating.

The vinyl or alk-1-enyl ether cyclocarbonates can be prepared directly by the addition of carbon dioxide to the corresponding vinyl or alk-I-eny glycidyl ether, in the presence of phase transfer catalysts, such as tetrabutyl ammonium bromide or iodide, tetraethyl ammonium bromide or iodide and cyclic ethers such as 18 crown 6 ether with potassium iodide. The reaction is generally run under pressure ranging from 344 to 6890 kPa (50 to 1000 psig) at a temperature of from 50°C. to 150°C. for a period of from one hour to 24 hours and the product isolated by distillation.

Preferably the cyclocarbonates are prepared by a two-stage liquid phase process wherein an alk-2-enyl glycidyl ether is catalytically isomerized to the corresponding alk-1-enyl glycidyl ether in the first-stage of the reaction and the reaction mixture of the first-stage, containing the isomerization catalyst, is then contacted with carbon dioxide as described.

The alk-2-enyl ether cyclocarbonates can also be prepared by reacting an alk-2-enyl ether diol with a dialkyl carbonate in the presence of a base catalyst such as sodium or potassium methoxide or butoxide, sodium or potassium hydroxide or sodium or potassium metal. As the reaction proceeds the evolving alkanol is distilled out of the system as an azeotrope along with some of the dialkyl carbonate and make-up dialkyl carbonate is continuously added. The temperature for this reaction can vary between about 70 and 100°C. and reaction time is typically between 1 and 8 hours. The product is then isolated by vacuum distillation. Alternatively the alk-2-enyl ether cyclocarbonates can be prepared by addition of carbon dioxide to alk-2-enyl glycidyl ethers using the conditions described above.

The alk-1-enyl ether cyclocarbonates, where R is lower alkyl, can be prepared by first synthesizing a alk-2-enyl ether cyclocarbonate as described above and isomerizing it to the corresponding alk-I-enyl ether Pure alk-2-enyl ether cyclocarbonate is catalytically isomerized to the corresponding alk-1-enyl ether by heating in the presence of known isomerization catalysts, such as RuCl,₉.H₂0, H₂Ru(PPh₃)₄, C1₂Ru(PPh₃)₄, rhodium chloride, palladium acetate, palladium on carbon or alumina,. or ruthenium on alumina or carbon. Isomerization temperatures range from about 100°C. to about 180°C. Higher than 99% isomerization can be achieved in one to several hours when a homogeneous catalyst is employed, while several hours to one day is required when a heterogeneous catalyst is used. Solvents are optionally used in this reaction.

Suitable cationic initiators include the onium salt initiators such as the triphenyl sulfonium salt of phosphorous hexafluoride, diphenyl iodium salt, tetrazolium chloride, phenyl onium salts or aryl alkyl onium salts. Mixtures of the present cationic initiators with up to 60% of free radical photoinitiators can be employed as the photoinitiator, if desired.

Suitable monomers and oligomers used in the above composition include the divinyl ether of tripropylene glycol, dimethyl cyclohexyl divinyl ether, hexanediol divinyl ether, hexadecyl monovinyl ether, vinyl ether terminated urethanes, diglycidyl ethers of bisphenol A, novalac epoxy resins, Epon resins, epoxy acrylate resins, acrylate urethane resins and oligomers of the above monomers as well as mixtures of the above monomers and/or oligomers.

The present compositions are easily prepared by forming a slurry of cationic initiator preferably in a concentration of from about 1 to about 3 wt. % in liquid component (b) or a solution of component (b). Component (a) in the desired amount, is slowly added to the slurry with continued stirring until a clear solution is obtained. This procedure is normally carried out under about ambient conditions; although elevated temperatures up to about 100°C. and pressure up to about 344 kPa (50 psig) can be employed when desired.

Optional adjuvants may be added to the composition to supply additional wetting characteristics. Suitable wetting agents include fluorinated alkyl ester surfactants, ethoxylates of alkyl phenols, fatty alcohols and alphaolefins.

The above composition are suitable for coating on a substrate such as glass, ceramic, wood, plastic, metal, leather, paper and the like in a thickness of from about 0.1 to about 5 mils.

Curing of the present compositions can be effected in less than 1 second by exposure to a source of radiation, e. g. between about 100 and about 800 millijoules/cm² of UV light, between about 0.5 and about 5 megarads of electron beam exposure or equivalent radiation exposures of gamma ray, X-ray, etc. Laser emissions can also be employed to effect curing.

The general techniques for exposing coating surfaces to radiation for curing are well known and the present compositions are cured in a conventional manner.

The alk-2-enyl glycidyl ether for use in the process of making the cyclocarbonates has the formula 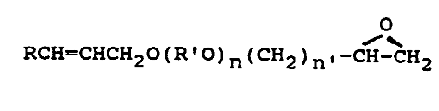 wherein R is hydrogen or lower alkyl, R' is C₂ to C₄ alkylene, n has a value of from 0 to 4 and n' has a value of from 1 to 4.

The first-stage reaction is carried out in an unpressurized system at a temperature of between about 100° and about 155°C.; although higher temperatures, e.g. up to about 180°C. can be employed in a system under pressure of up to about 344 kPa (50psi). The isomerization reaction is completed within a period of from about 2 to about 20 hours. Preferred conditions include reflux temperature under atmospheric pressure for 5 to 7 hours, during which greater than 98% yield of the alk-1-enyl glycidyl ether isomeric product is produced. The entire reaction mixture from the first-stage is then treated with carbon dioxide gas in the second-stage at a temperature of from about 100° to about 150°C. under a pressure of from about atmospheric to about 400 psig for a period of between about 1 and about 48 hours after which the alk-1-enyl ether cyclocarbonate is recovered in greater than 98% yield. Preferred conditions for the second-stage reaction include a temperature of between about 115° and about 135°C. under carbon dioxide pressure of from 14 to 1380 kPa (2 psi to 200 psig) for a period of 8 to 15 hours.

The first-stage reaction is carried out in the presence of between 0.001 and 1 wt. %, preferably between 0.01 and 0.1 wt. %, of a homogeneous or heterogeneous isomerization catalyst. Suitable homogeneous isomerization catalysts include ruthenium trichloride hydrate, ruthenium dioxide, triruthenium dodecarbonyl, ruthenium acetylacetonate, palladium chloride, palladium acetate, rhodium chloride, and the like Suitable heterogeneous isomerization catalysts include supported ruthenium, palladium, or rhodium metal on alumina, carbon or silicas as the support.

The second-stage addition reaction is effected in the presence of between 0.005 and 5 wt. %, preferably between 0.3 and 3 wt. %, of a phase transfer catalyst such as a tetraalkyl ammonium halide, e.g. tetrabutyl ammonium bromide, tetraethyl ammonium iodide, a cyclic ether such as 18 crown 6 ether in conjunction with a potassium halide, e.g. iodide or bromide, or a bicyclic amino ether or bicyclic amine, such as triethylenediamine, with potassium iodide

The combination of the isomerization and phase transfer catalysts in the second-stage reaction eliminates the need for multiple distillation operations since, in the present process, distillation is required only after completion of the second stage reaction to obtain greater than 98% product purity Additionally, the combination of these two catalyst systems permits faster conversion to the cyclocarbonate product which is recovered in high purity

The crude alk-1-enyl ether cyclocarbonate can be purified by any convenient method including flashing at reduced pressure, e.g. in a wiped film evaporator under 5-10 mm Hg. Any other purification method, e.g. carbon treatment, and/or water washing can be employed when additional product purification is desired.

The final product is a mixture of cis and trans alk-I-enyl ether cyclocarbonates containing less than 2% impurity

### EXAMPLE 1

Into a one-liter stainless steel autoclave is introduced 500 grams of allyl glycidyl ether (supplied by Alcolac Chemical Company) and 0.05 wt. % of ruthenium chloride hydrate. The resulting reaction mixture was heated to 154°C. for 6 hours after which greater than 98% yield of the corresponding prop-I-enyl glycidyl ether is produced. To this mixture at 125°C. is added 0.5 wt. % tetrabutyl ammonium bromide. Carbon dioxide is then pressured into the resulting mixture under a pressure of 2067 kPa (300 psig) for 12 hours, after which greater than 98% yield of the desired cis and trans mixture of the prop-1-enyl ether of propylene carbonate is produced This product is treated in a wiped film evaporator at a temperature of 130°C. under 2 mm Hg (blade rpm 325) at a rate of 500 cm³ per hour, to produce the product in greater than 98% purity. A total yield of 92% is obtained.

### EXAMPLE 2

A. To a 4 dram vial containing 2.5 g. of liquid divinyl ether of triethylene glycol (RAPICURE DVE-3 ⁽¹⁾) was added 5 ml of FX-512(²), i.e. a mixture of aryl sulfonium salts of hexafluorophosphate. The 20% solution was then blended with RAPICURE DVE-3 and RAPICURE CHVE, (i.e. the divinyl ether of 1,4-cyclohexane dimethanol) (³) to reduce the FX-512 initiator concentration to 2%. The percent of RAPICURE DVE-3 required to solubilize 2% initiator in RAPICURE CHVE is reported in following Table I.

B. Part A was repeated except that the prop-I-enyl ether of propylene carbonate (PEPC) was substituted for RAPICURE DVE-3. The percent of PEPC required to solubilize 2% initiator in RAPICURE CHVE is also reported in following Table I.

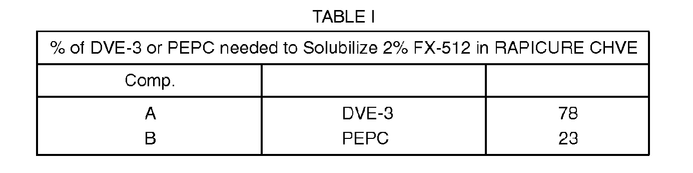

Upon curing coatings using EPON-828 (diepoxide of bisphenol A resin) as the polymerizable oligomer in above systems A and B, a yellowish product was produced by coating A whereas no color development occurred with coating B.

### EXAMPLE 3

To a 4 ounce amber colored bottle was added 25 g. of vinyl ether terminated urethane oligomer i.e. Vectomer 2020 (⁴) and 25 g. of RAPICURE CHVE at a temperature of 45°C. with constant agitation. After a uniformly blended liquid mixture was obtained, 0.25 g. of a fluorinated alkyl ester surfactant (Fluorad 171⁽²⁾) and 2 g. of FX-512 initiator were added and mixed therein at 45°C. The resultant formulation is designated Sample 2.
(1) Supplied by GAF Chemicals Corp.
(2) Supplied by Minnesota Mining and Mfg. Co.
(3) Supplied by GAF Chemicals Corporation
(4) Supplied by Allied-Signal Corp.

### EXAMPLE 4

The procedure of Example 3 was repeated except that 5 g. of PEPC was substituted for 5 g. of the 25 g. of RAPICURE CHVE. The resultant formulation is designated Sample 3.

### EXAMPLE 5

The procedure of Example 3 was repeated except that 5 g. of RAPICURE DVE-3 was replaced for 5 g. of RAPICURE CHVE. The resultant liquid is designated Sample 4.

### EXAMPLE 6

To a 4 ounce amber colored bottle was added 25 g. of acrylate terminated epoxy oligomer such as Ebacryl 6700 from RadTech Specialties, 20 g. of RAPICURE CHVE and 5 g. of PEPC at temperature of 45° with constant agitation. After a uniformly blended liquid was obtained, a hybrid initiator mixture of 1 g. of FX-512 cationic photoinitiator and phenyl-1-hydroxy cyclohexane ketone free radical initiator (Irgacure 184, supplied by Ciba-Giegy), together with 0.25 g. of Fluorad 171, were added and mixed at 45° for 20 minutes. The resultant formulation was designated as Sample 5.

### EXAMPLE 7

To a 4 ounce amber colored bottle was added 25 g. of bisphenol A diepoxide having a molecular weight of 500 to 560, Epon 1001, 20 g. RAPICURE CHVE and 5 g. of PEPC at a temperature of 45°C. with constant agitation. After a uniformly blended liquid is obtained, 0.25 g. of Fluorad 171 and 1 g. of FX-512 were added and mixed at 45°C. for 20 minutes. The resultant formulation was designated as Sample 6.

### EXAMPLE 8

To a 4 ounce amber colored bottle was added 25 g. of bisphenol A diepoxide having a molecular weight of 500 to 560, Epon 1001 and 25 g. of RAPICURE CHVE at a temperature of 45°C. with constant agitation. After a uniformly blended liquid is obtained, 0.25 g of Fluorad 171 and 1 g. of FX-512 were added and mixed at 45°C for 20 minutes. The resultant formulation was designated as Sample 7.

### EXAMPLE 9

To a 4 ounce amber colored bottle was added 25 g. of bisphenol A diepoxide having a molecular weight of 370 to 384, Epon 825 and 25 g. of RAPICURE CHVE at a temperature of 45°C. with constant agitation. After a uniformly blended liquid is obtained, 0.25 g. of Fluorad 171 and 1 g. of FX-512 were added and mixed at 45°C. for 20 minutes. The resultant formulation was designated as Sample 8.

### EXAMPLE 10

The procedure for Example 9 was repeated except that 5 g. of RAPICURE CHVE was replaced with 5 g. of PEPC. The resultant formulation was designated as Sample 9.

Each of the above samples were examined for clarity of the solution Samples 2, 4 and 7 were cloudy; whereas Sample 3, 5, 6, 8 and 9 were clear. These samples were individually coated on aluminum panels by hand draw downs using a 12 Mayer bar to give a coating thickness of about 1.0 microns. The corresponding panels 2-9 were then subjected to a UV light exposure at 15 joules/cm² by passing them under two 78 W/cm² (200 watt/inch) medium pressure mercury vapor lamps at 472 cm/mn (100 feet/minute) and then tested for solvent resistance, release impact and elongation.

For the solvent resistance test, a methylethyl ketone saturated cheesecloth was rubbed across the surface of the coated panel under a constant pressure. The number of back and forth strokes needed to break through the coating was recorded. The reverse impact test was carried out by placing a coated panel face down on a die containing a 1.63 cm (0.640 inch) hole. A 1.59 cm (0.625 inch) pin with rounded tip was placed on the back of the panel directly over the hole. A 0.45 kg (1 Ib) weight was dropped, from varying heights, onto the pin causing rapid deformation of the panel and coating and the coating was examined for cracking or crazing. The maximum energy the coating can absorb before failure was recorded. The results of these tests are reported in following Table II.

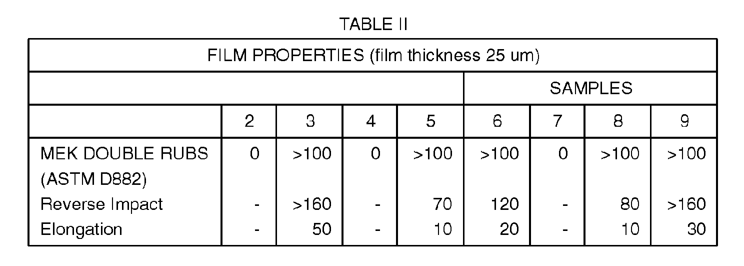

It is to be understood that other reactive diluents of this invention can be substituted in Example 4 or in Example 7 or in Example 9 to provide clear initiator solutions and cured compositions which are chemically resistant. Specifically the vinyl ether substituted cyclocarbonates provide excellent results.

## Claims

1. A coating composition rapidly curable by exposure to a source of radiation which comprises a cationically copolymerizable mixture of
(a) between 1 and 75 wt. % of a reactive vinyl or alk-1-enyl ether cyclocarbonate having the formula 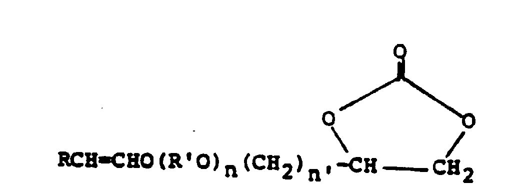 wherein R is hydrogen or lower alkyl; R' is C₂ to C₄ alkylene; n has a value of from 0 to 4 and n' has a value of from 1 to 4 and
(b) between 99 to 25 wt. % of a polymerizable compound containing between 1 and 5 wt. % of a cationic onium salt initiator which is solubilized by (a).

2. The coating composition of claim 1 wherein n' has a value of one and n is zero.

3. The coating composition of claim 1 wherein said onium salt initiator is the triphenyl sulfonium salt of phosphorous hexafluoride.

4. The coating composition of claim 1 wherein said polymerizable compound is a monomer or oligomer selected from the group of a vinyl ether, an alk-1-enyl ether, an epoxide, an acrylate, a methacrylate, a vinyl ether urethane, a vinyl lactam or a combination thereof

5. The coating composition of claim 1 wherein said component (b) is divinyl ether of tripropylene glycol, 1,4-dimethylcyclohexyl divinyl ether, or a mixture of 1,4-dimethylcyclohexane and a vinyl ether terminated urethane oligomer.

6. The coating composition of claim 1 wherein component (a) is 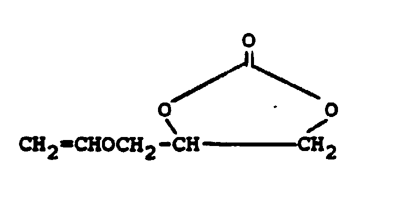 or

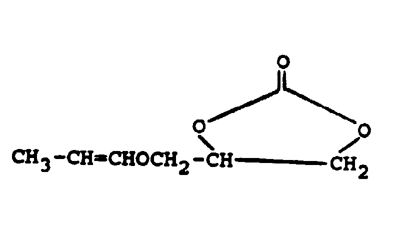

7. A two-stage process for synthesizing an alk-1-enyl ether cyclocarbonate of claim 1 from an alk-2-enyl glycidyl ether which comprises isomerizing an alk-2-enyl glycidyl ether in the presence of between 0.001 and 1 wt. % of an isomerization catalyst at a temperature of between 100°C. and 180°. under a pressure of from about atmospheric to about 344 kPa (50 psig) to produce a catalyst/alk-1-enyl glycidyl ether mixture as an intermediate product and then reacting said mixture with carbon dioxide in the presence of between about 0.005 and about 5 wt. % of a phase transfer catalyst at a temperature of between about 100°C. and about 150°C. under a pressure of from about atmospheric to about 2756 kPa (400 psig) to produce the corresponding alk-1-enyl ether cyclocarbonate as the product of the process.

8. The process of claim 7 wherein said isomerization catalyst is a homogeneous or a heterogeneous catalyst selected from the group of ruthenium dioxide, ruthenium trichloride hydrate, triruthenium dodecarbonyl, ruthenium acetylacetonate, palladium chloride, palladium acetate, rhodium chloride, ruthenium, palladium or rhodium metal supported on alumina carbon or silica and the phase transfer catalyst is selected from the group of a tetraalkyl ammonium halide, a cyclic crown ether in conjunction with potassium iodide, potassium bromide or a bicyclic amino ether or bicyclic amine in conjunction with potassium iodide or bromide.

## Patentansprüche

1. Beschichtungszusammensetzung, die durch Einer-Strahlungsquelle-Aussetzen rasch härtbar ist und ein kationisch copolymerisierbares Gemisch aus
(a) zwischen 1 und 75 Gew-% eines reaktiven Vinyl- oder Alk-1-enylethercyclocarbonats mit der Formel 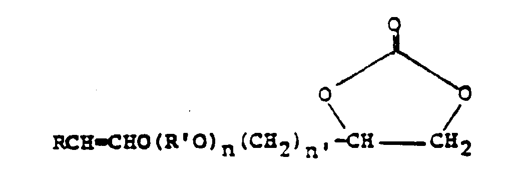 worin R Wasserstoff oder Niederalkyl ist; R' C₂- bis C₄-Alkylen ist; n einen Wert von 0 bis 4 und n' einen Wert von 1 bis 4 besitzt, und
(b) zwischen 99 und 25 Gew.-% einer polymerisierbaren Verbindung, die zwischen 1 und 5 Gew.-% eines kationischen Oniumsalzinitiators enthält, der durch (a) solubilisiert wird,
umfaßt.

2. Beschichtungszusammensetzung nach Anspruch 1, worin n' einen Wert von 1 besitzt und n 0 ist.

3. Beschichtungszusammensetzung nach Anspruch 1, worin der Oniumsalzinitiator das Triphenylsulfoniumsalz von Phosphorhexafluorid ist.

4. Beschichtungszusammensetzung nach Anspruch 1, worin die polymerisierbare Verbindung ein Monomer oder Oligomer, ausgewählt aus der Gruppe bestehend aus einem Vinylether, einem Alk-1-enylether, einem Epoxid, einem Acrylat, einem Methacrylat, einem Vinyletherurethan und einem Vinyllactam, oder eine Kombination davon ist

5. Beschichtungszusammensetzung nach Anspruch 1, worin die Komponente (b) Divinylether von Tripropylenglykol, 1,4-Dimethylcyclohexyldivinylether oder ein Gemisch aus 1,4-Dimethylcyclohexan und einem Urethanoligomer mit Vinylether-Endgruppe ist.

6. Beschichtungszusammensetzung nach Anspruch 1, worin Komponente (a) folgende Formeln aufweist: 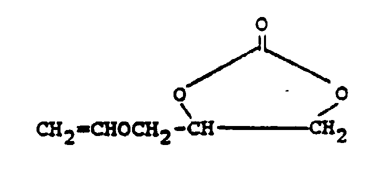 oder

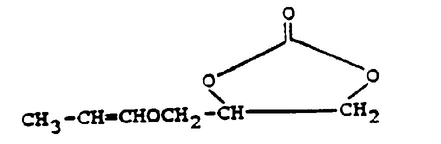

7. Zweistufiges Verfahren zum Synthetisieren eines Alk-1-enylethercyclocarbonats nach Anspruch 1 aus einem Alk-2-enylglycidylether, welches das Isomerisieren eines Alk-2-enylglycidylethers in Gegenwart von zwischen 0,001 und 1 Gew.% eines Isomerisierungskatalysators bei einer Temperaturvon zwischen 100°C und 180°C unter einem Druck von etwa atmosphärischem Druck bis etwa 344 kPa (50 psig), um ein Gemisch aus Katalysator und Alk-1-enylglycidylether als Zwischenprodukt zu bilden, und anschließend das Umsetzen dieses Gemisches mit Kohlendioxid in Gegenwart von zwischen etwa 0,005 und etwa 5 Gew.-% eines Phasentransferkatalysators bei einer Temperatur von zwischen etwa 100°C und etwa 150°C unter einem Druck von etwa atmosphärischem Druck bis etwa 2756 kPa (400 psig), um das entsprechende Alk-1-enylethercyclocarbonat als Produkt des Verfahrens herzustellen, umfaßt.

8. Verfahren nach Anspruch 7, worin der Isomerisierungskatalysator ein homogener oder ein heterogener Katalysator ist, ausgewählt aus der Gruppe bestehend aus Rutheniumdioxid, Rutheniumtrichloridhydrat, Trirutheniumdodecarbonyl, Rutheniumacetylacetonat, Palladiumchlorid, Palladiumacetat, Rhodiumchlorid, Ruthenium-, Palladium-oder Rhodiummetall, auf Aluminiumoxid, Kohlenstoff oder Quarz als Trägermaterial, und worin der Phasentransferkatalysator aus der Gruppe bestehend aus einem Tetraalkylammoniumhalogenid, einem zyklischen Kronenether in Verbindung mit Kaliumiodid, Kaliumbromid oder einem bizyklischen Aminoether oder einem bizyklischen Amin in Verbindung mit Kaliumiodid oder -bromid ausgewählt ist.

## Revendications

1. Composition de revêtement rapidement polymérisable par exposition à une source d'irradiation qui comprend un mélange copolymérisable cationiquement de
(a) entre 1 et 75 % en poids d'un vinyl alk-1-ènyl éther cyclocarbonate réactif ayant la formule 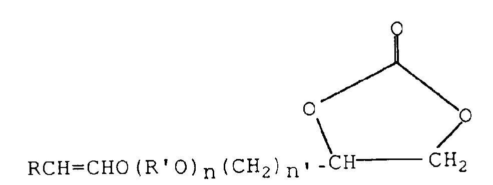 où R est un hydrogène ou un alkyle inférieur ; R' est un alkylène en C₂ à C₄ ; n a une valeur de 0 à 4 et n' a une valeur de 1 à 4 et
(b) entre 99 à 25 % en poids d'un composé polymérisable contenant entre 1 et 5 % en poids d'un initiateur sel onium cationique qui est solubilisé par (a).

2. Composition de revêtement selon la revendication 1 ou n'a a une valeur de 1 et n vaut zéro.

3. Composition de revêtement selon la revendication 1 dans laquelle l'initiateur sel onium est le sel triphényl sulfonium de l'hexafluorure phosphoreux.

4. Composition de revêtement selon la revendication 1 où ledit composé polymérisable est un monomère ou un oligomère choisi dans le groupe d'un vinyl éther, d'un alk-1-ènyl éther, d'un époxyde, d'un acrylate, d'un méthacrylate, d'un vinyl éther uréthane, d'un vinyl lactam ou d'une composition de ceux-ci.

5. Composition de revêtement selon la revendication 1 où ledit composant (b) est le divinyl éther de tripropylène glycol, le 1,4 diméthylcyclohexyl divinyl éther, ou un mélange de 1,4 diméthylcyclohexane et d'un oligomère uréthane terminé par un vinyl éther.

6. Composition de revêtement selon la revendication 1 où le composant (a) est 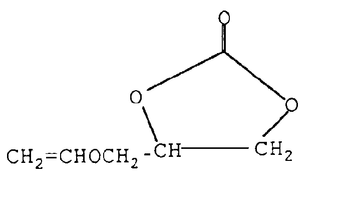 ou

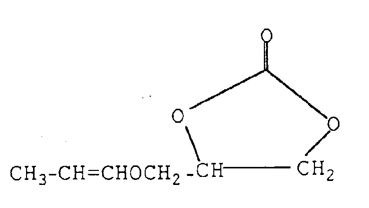

7. Procédé en deux étapes pour synthétiser un alk-1-ènyl éther cyclocarbonate selon la revendication 1 à partir d'un alk-2-ènyl glycidyl éther qui comprend l'isomérisation d'un alk-2-ènyl glycidyl éther en présence d'entre 0,001 et 1 % en poids d'un catalyseur d'isomérisation à une température d'entre 100°C et 180°C sous une pression de l'atmosphère à environ 344 kPa (50 psig) pour produire un mélange catalyseur/alk-1-ényl glycidyl éther en tant que produit intermédiaire et ensuite la réaction dudit mélange avec du dioxyde de carbone en présence d'entre environ 0,005 et environ 5 % en poids d'un catalyseur de transfert de phase à une température d'entre environ 100°C et environ 150°C sous une pression d'environ l'atmosphère à environ 2756 kPa (400 psig) pour produire l'alk-1 ènyl éther cyclocarbonate correspondant en tant que produit du procédé

8. Procédé selon la revendication 7 dans lequel ledit catalyseur d'isomérisation est un catalyseur homogène ou hétérogène choisi dans le groupe du ruthénium dioxyde, ruthénium trichlorhydrate, de triruthénium dodécarbonyle, du ruthénium acétylacétonate, du palladium chlorure, du palladium acétate, du rhodium chlorure, du ruthénium, palladium ou rhodium métalliques supportés sur de l'alumine charbon ou de la silice et le catalyseur de transfert de phase est choisi dans le groupe d'un tétraalkyl ammonium halogénure, d'un éther couronne cyclique en conjonction avec du potassium iodure, du potassium bromure ou un amino éther bicyclique ou une amine bicyclique en conjonction avec du potassium iodure ou bromure.
